# EUROPÄISCHE PATENTSCHRIFT

(11) **EP 2 536 681 B1**
(45) Veröffentlichungstag und Bekanntmachung des Hinweises auf die Patenterteilung: **03.08.2016**
(21) Anmeldenummer: 11703014.8
(22) Anmeldetag: 15.02.2011
(51) Int. Cl.: C07C 51/295, C07C 59/305, C07C 51/235, C07C 227/02

(54) **VERFAHREN ZUR HERSTELLUNG VON ETHERCARBOXYLATEN**
METHOD FOR THE PRODUCTION OF ETHER CARBOXYLATES
PROCÉDÉ DE PRÉPARATION D'ÉTHER CARBOXYLATES

(30) Priorität: 19.02.2010 EP 10154071
(43) Veröffentlichungstag der Anmeldung: 26.12.2012
(73) Patentinhaber: BASF SE, 67056 Ludwigshafen am Rhein (DE)
(72) Erfinder: BAUMANN, Robert, 68529 Mannheim (DE); BIEL, Markus, Christian, 68159 Mannheim (DE); DECKERS, Andreas, 55234 Flomborn (DE); OFTRING, Alfred, 67098 Bad Dürkheim (DE); RITTIG, Frank, 67549 Worms (DE); STAFFEL, Wolfgang, 67166 Otterstadt (DE)
(74) Vertreter: BASF IP Association
(86) Internationale Anmeldenummer: PCT/EP2011/052191
(87) Internationale Veröffentlichungsnummer: WO 2011/101336

(56) Entgegenhaltungen:
- EP-A1- 0 620 209
- EP-A1- 1 382 390
- EP-A2- 0 945 428
- WO-A1-98/13140
- DE-A1- 3 135 946
- US-A- 2 183 853
- US-A- 3 717 676
- US-A- 3 799 977
- US-A- 3 935 257
- US-A- 4 110 371
- US-A1- 2003 097 020

## Beschreibung

Die vorliegende Erfindung betrifft ein Verfahren zur Herstellung von Ethercarboxylaten. Ethercarboxylate werden üblicherweise absatzweise hergestellt, wobei normalerweise das Alkoxylat, eine Base wie NaOH und ein Katalysator wie Raney-Kupfer vorgelegt werden und diese Reaktionsmischung unter Druck auf Reaktionstemperatur gebracht wird.

Die Klasse der Ethercarboxylate als milde anionische Tenside ist bereits seit langem bekannt. Bereits US 2,183,853 aus dem Jahre 1934 beschreibt die Herstellung von Ethercarboxylaten durch Umsetzung von Alkoxylaten mit Natrium und Chloressigsäurenatriumsalz bei Temperaturen zwischen 160 und 200 °C. Um hohe Umsätze und geringe Nebenproduktbildung zu erreichen, wird es als vorteilhaft beschrieben, die Base und Chloressigsäure unter Rühren an verschiedenen Stellen des Reaktors einzudosieren. Um entstehendes bzw. zugeführtes Wasser aus der Reaktionslösung zu entfernen und um eine möglichst schnelle Reaktion zu erreichen, wird die Umsetzung bei 70 bis 90 °C und einem Druck von ca. 10 bis 50 mbar durchgeführt.

DE 31 35 946 beschreibt als weitere Variante zur Herstellung von Ethercarboxylaten die direkte Oxidation der Alkoxylate mit Sauerstoff oder Sauerstoff enthaltenden Gasen in wässrig alkalischem Medium an einem Platin- bzw. Palladiumkatalysator.

Die oxidative Dehydrierung von Alkoholen zu Carboxylaten durch thermische Umsetzung des Alkohols mit Alkalimetallen oder Alkalimetallhydroxiden ist bereits seit 1840 bekannt (Dumas und Stag, Ann, 35, 129 bis 173). Diese Umsetzung wurde klassisch ohne Katalysator und bei Temperaturen > 200 °C durchgeführt. In US 2,384,818 aus dem Jahr 1945 wurde diese Methode zur Oxidation von Aminoalkoholen bereits angewendet.

Auch die Verwendung von Katalysatoren für die oxidative Dehydrierung ist seit langem bekannt. US 2,384,817 aus dem Jahr 1942 beschreibt den positiven Einfluss von Cadmium, Kupfer, Nickel, Silber, Blei und Zinkverbindungen auf die Reaktionsgeschwindigkeit.

EP 0 620 209 beschreibt einen Prozess zur Herstellung von Carbonsäuresalzen durch in Kontakt bringen einer wässrigen Lösung eines primären Alkohols mit einem Alkalimetallhydroxid in Anwesenheit einer wirksamen Menge eines speziellen und aktivierten Raney-Kupfer-Katalysators, der 50 bis 10.000 ppm eines Elements aus Chrom, Titan, Niob, Tantal, Zirkonium, Vanadium, Molybdän, Mangan, Wolfram, Kobalt und Mischungen hiervon enthält oder 50 bis 10.000 ppm Nickel. Der Alkohol kann aliphatisch, aromatisch oder ein Polyol sein. Es wird beschrieben, dass sowohl der Alkohol - der sich notwendigerweise durch eine gewisse Wasserlöslichkeit auszeichnet - als auch das entstehende Carboxylat in der heißen und ätzenden Lösung stabil sein müssen. Auβerdem ist für die Reaktion Druck notwendig, damit die beschriebenen Reaktionstemperaturen erreicht werden können. Als Beispiele sind Aminoalkohole, aromatische Alkohole und Ethylenglykol aufgeführt.

EP 1 125 633 beschreibt die Verwendung von dotiertem Raney-Kupfer (Dotierung mit mindestens einem Element aus der Eisengruppe und oder einem Edelmetall), dessen Inaktivierung durch Agglomeration deutlich geringer und die Verwendungscyclen daher höher sind. Die Erfindung betrifft die Herstellung von Carbonsäuren aus Alkoholen. Als Beispiel wird auch hier die oxidative Dehydrierung von Diethanolamin unter stark basischen Bedingungen bei 160 °C und 10 bar Druck angeführt. Die Alkohole müssen in Wasser löslich und der Alkohol sowie die Carbonsäure gegenüber der stark basischen Lösung stabil sein.

EP 1 125 634 beschreibt einen Raney-Kupfer-Festbettkatalysator (mit Dotierung aus Eisen bzw. Edelmetall), dessen einfache Filtrierbarkeit von der Reaktionslösung und dessen Verwendung im Rahmen eines kontinuierlichen Prozesses. Als Voraussetzung für die Umsetzung der Alkohole wird beschrieben, dass sowohl Edukt als auch Produkt in den stark basischen Lösungen stabil sein müssen und der Alkohol eine Wasserlöslichkeit aufweisen muss.

US 5,916,840 beschreibt zur Herstellung von Carbonsäuresalzen einen geträgerten Katalysator bestehend aus einem alkalistabilen Trägermaterial (z. B. Aktivkohle) einem Ankermetall (z. B. Palladium) und einem punktuell aufgetragenen Aktivmetall (z. B. Kupfer). Die Umsetzung findet im batch-Autoklaven unter Wasserzusatz bei 160 bis 170 °C statt. In Fortführung dieses Prinzips beschreibt WO 03/033140 weitere Katalysatoren zur Herstellung von Iminodiessigsäure ausgehend von Diethanolamin. Als bevorzugte Reaktionsbedingung wird die Verwendung von Alkalimetallhydroxiden (äquimolarer Einsatz), Nutzung eines Lösungsmittels und eines Katalysators (mindestens 1 Massen%) bei Temperaturen von 120 bis 220 °C unter Druck genannt.

WO 98/13140 beschreibt einen Katalysator zur Dehydrierung von Aminoalkoholen und Ethylenglykolderivaten, bestehend aus Zirkonium, Kupfer und gegebenenfalls einem weiteren Metall. Es wird die Umsetzung von Ethylenglykolen wie Triethylenglykol mit 1eq NaOH in wässriger Lösung bei 180 °C und 10 bar Druck am beschriebenen Katalysator (30 Massen%) offenbart.

US 4,110,371 beschreibt ein Verfahren zur Herstellung von Dicarbonsäuresalzen ausgehend von Verbindungen bestehend aus 2 bis 6 Ethylenglykoleinheiten mit wässriger Alkalilauge bei Temperaturen von 200 bis 300 °C unter Verwendung eines Katalysators bestehend aus Nickel, Kupfer und Chrom.

US 3,717,676 beschreibt ein Verfahren zur Herstellung von Alkalisalzen von Oxypolycarboxylaten in dem ein oxysubstituierter oder polyoxysubstituierter primärer Alkohol mit Alkalimetallhydroxid, 20 bis 60 % Wasser bei 190 bis 230 °C, einem Druck von 7 bis 14 bar und einem Cadmium-Katalysator umgesetzt wird.

JP 10 277 391 schließlich beschreibt die Verwendung von ultra-feinen Kupfer-Katalysatoren mit Korngrößen von 1 bis 20 microns zur Herstellung von Alkylethercarboxylaten ausgehend von Polyoxyethylenalkylethern und deren Verwendung als anionisches Tensid in Seifen- und Kosmetik-Anwendungen. Die feine Katalysatorverteilung zeigt gegenüber klassischen Raney-Kupfer bzw. Kupfer-Zirkonium-Katalysatoren eine deutlich höhere Aktivität (Vergleichsbeispiel mit Raney-Kupfer von Degussa zeigt unter ansonsten äquivalenten Bedingungen lediglich einen Umsatz von 15 statt 98%).

All die beschriebenen Verfahren weisen demnach eine Reihe von Nachteilen auf, die trotz der langen Zeit, die auf diesem Gebiet bereits geforscht worden ist, nicht überwunden werden konnten. So ist immer noch unter einer Kombination aus Druck, hohen Temperaturen und stark alkalischen Bedingungen zu arbeiten, was das verwendete Material stark belastet (Spannungsrisskorrosion), die Reaktivität ist ebenso begrenzt wie die Selektivität, die verwendeten Katalysatoren sind teuer, da sie aufwändig dotiert werden müssen und verklumpen während des Verfahrens, es sind nur wasserlösliche und somit häufig eher kurzkettige Alkohole verwendbar, zudem müssen sowohl verwendeter Alkohol als auch entstehendes Produkt basenstabil sein und Vinylether, die häufig als Spaltprodukte entstehen, sind im entstehenden Produkt nicht erwünscht dabei aber analytisch nur schlecht nachweisbar. Es ist daher Aufgabe der vorliegenden Erfindung ein Verfahren bereitzustellen, das die genannten Nachteile verringert bzw. beseitigt.

Es wurde überraschend gefunden, dass ein Verfahren zur Herstellung von Ethercarboxylat der Formel I 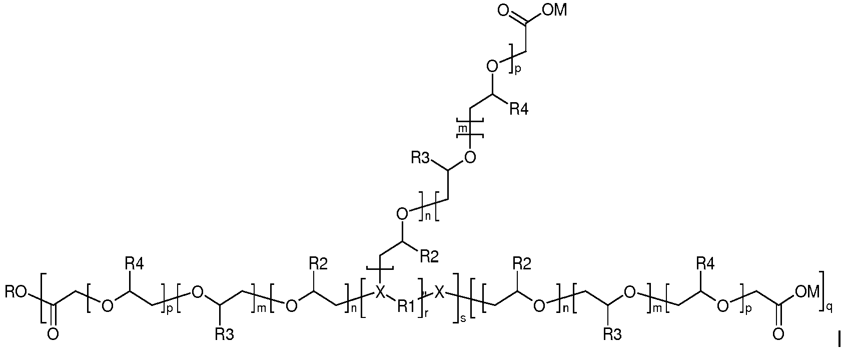 bei dem das entsprechende Alkoxylat der Formel II 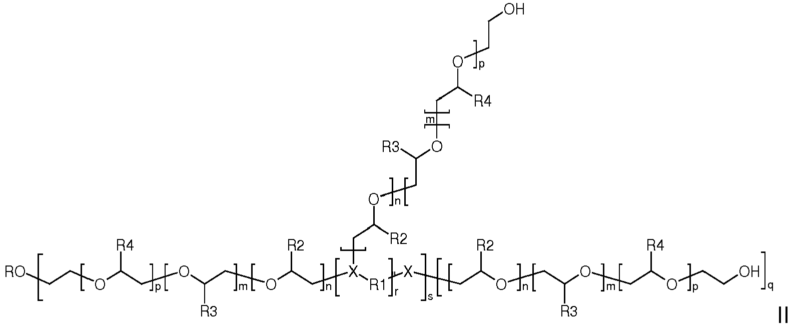 wobei jeweils unabhängig voneinander
R1 ein C₁- bis C₅₀-Alkyl, Mono-, Di-, Tri-,...Polyamin,
X O, COO, CH₂-NH-O für r = 0 und q = 1 oder N, 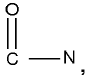 N(CH₂)ₜO für r = 0 bis 50 und q = 2,
r eine ganze Zahl von 0 bis 50,
R2 H oder C₁- bis C₁₀-Alkyl,
R3 H oder ein C₁- bis C₁₀-Alkyl,
R4 H oder ein C₁- bis C₁₀-Alkyl,
M H oder ein Metall, bevorzugt Alkali, Erdalkali, Ammonium, organische Base,
n eine Zahl von 0 bis 50,
m eine Zahl von 0 bis 40 ist,
p eine Zahl von 0 bis 40 ist,
q eine Zahl von 1 bis 20 ist,
s 0 oder 1 ist,
t 0 bis 20 ist,
mit der Maßgabe, dass n + m + p mindestens 1 ist und
R ein C₁- bis C₅₀-Alkyl, C₁- bis C₅₀-Alkenyl, C₁- bis C₅₀- Alkinyl, C₆- bis C₅₀-Aryl,
C₆- bis C₅₀-Alkylaryl H oder ein Metall, bevorzugt Alkali, Erdalkali istmit einer Base unter Verwendung eines Übergangsmetallkatalysators, der als Raney-Kupfer vorliegt, umgesetzt wird und wobei der Druck im Reaktionsgefäß weniger als 10 bar beträgt, die gestellte Aufgabe löst.

Es gibt verschiedene bevorzugte Ausführungsformen, wobei in der einen, wenn s 0 ist, R ein C₁- bis C₅₀-Alkyl, C₁- bis C₅₀-Alkenyl, C₁- bis C₅₀- Alkinyl, C₆- bis C₅₀-Aryl oder C₆- bis C₅₀-Alkylaryl ist und in der anderen, wenn s 1 ist, R H oder ein Metall, bevorzugt Alkali, Erdalkali ist.

Auch gibt es bevorzugte Bereiche für r. Es weist vorzugsweise Werte von 1 bis 20 und besonders bevorzugt von 2 bis 10 auf.

Bevorzugt ist ein Verfahren zur Herstellung von Ethercarboxylat der Formel III 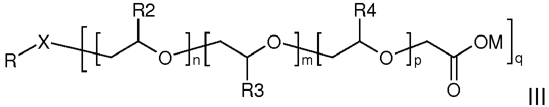 bei dem das entsprechende Alkoxylat der Formel IV 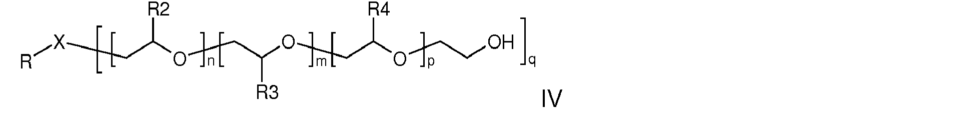 wobei jeweils unabhängig voneinander
R ein C₁- bis C₅₀-Alkyl, C₁- bis C₅₀-Alkenyl, C₁- bis C₅₀- Alkinyl, C₆- bis C₅₀-Aryl, C₆- bis C₅₀-Alkylaryl, H oder ein Metall, bevorzugt Alkali, Erdalkali ist,
X O, COO, CH₂-NH-O für q = 1 oder N, 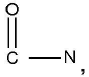 N(CH₂)ₜO für q = 2,
R2 H oder C₁- bis C₁₀-Alkyl,
R3 H oder ein C₁- bis C₁₀-Alkyl,
R4 H oder ein C₁- bis C₁₀-Alkyl,
M H oder ein Metall, bevorzugt Alkali, Erdalkali, Ammonium, organische Base,
n eine Zahl von 0 bis 50,
m eine Zahl von 0 bis 40 ist,
p eine Zahl von 0 bis 40 ist,
q eine Zahl von 1 bis 2 ist,
t eine Zahl von 1 bis 10 ist,
mit der Maßgabe, dass n + m + p mindestens 1 ist
mit einer Base unter Verwendung eines Übergangsmetallkatalysators, der als Raney-Kupfer vorliegt, umgesetzt wird und wobei der Druck im Reaktionsgefäß weniger als 5 bar beträgt.

Ein Verfahren zur Herstellung von Ethercarboxylat der Formel V 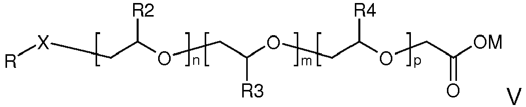 bei dem das entsprechende Alkoxylat der Formel VI 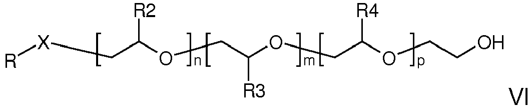 wobei jeweils unabhängig voneinander R ein C₁- bis C₅₀-Alkyl, C₁- bis C₅₀-Alkenyl, C₁- bis C₅₀- Alkinyl, C₆- bis C₅₀-Aryl, C₆- bis C₅₀-Alkylaryl, H oder ein Metall, bevorzugt Alkali, Erdalkali ist
X O ist,
R2 H oder C₁- bis C₁₀-Alkyl,
R3 H oder ein C₁- bis C₁₀-Alkyl,
R4 H oder ein C₁- bis C₁₀-Alkyl,
n eine Zahl von 0 bis 50,
m eine Zahl von 0 bis 40 ist,
p eine Zahl von 0 bis 40 ist, vorzugsweise von 1 bis 20, besonders bevorzugt von 5 bis 10,
mit der Maßgabe, dass n + m + p mindestens 1 ist
mit einer Base unter Verwendung eines Übergangsmetallkatalysators, der als Raney-Kupfer vorliegt, umgesetzt wird und wobei der Druck im Reaktionsgefäß weniger als 2 bar beträgt

In Bezug auf die eingesetzten Alkoxylate gibt es bevorzugte Varianten, so ist ein Verfahren bevorzugt, bei dem unabhängig voneinander
R ein C₁- bis C₅₀-Alkyl, C₁- bis C₅₀-Alkenyl, C₁- bis C₅₀- Alkinyl, C₆- bis C₅₀-Aryl, C₆- bis C₅₀-Alkylaryl, H oder ein Metall, bevorzugt Alkali, Erdalkali ist
X O ist,R2 H oder ein C₂- bis C₈-Alkyl,
R3 H oder ein C₂- bis C₈-Alkyl,
R4 H oder ein C₂- bis C₈-Alkyl,
n eine Zahl von 1 bis 30,
m eine Zahl von 1 bis 20 ist,
p eine Zahl von 1 bis 20 ist,
n + m + p mindestens 2 ist.

Besonders bevorzugt ist ein Verfahren, bei dem unabhängig voneinander
X O, COO
R ein C₂- bis C₁₀-Alkyl,
R2 H oder ein C₃- bis C₅-Alkyl,
R3 H oder ein C₃- bis C₆-Alkyl,
R4 H oder ein C₃- bis C₆-Alkyl,
n eine Zahl von 1 bis 18,
m eine Zahl von 1 bis 12 ist,
m eine Zahl von 1 bis 12 ist,
n + m + p mindestens 5 ist.

R ist dabei insbesondere Methyl, Ethyl, Propyl, Butyl, Pentyl, Hexyl, Octyl, 2-Ethylhexyl und 2-Propylheptyl, Dodecyl, Tridecyl, Mystryl, Lauryl, i-C17, Tallölfett (C16,18), Behenyl.

R2, R3, R4 sind dabei, soweit vorhanden, unabhängig voneinander insbesondere Wasserstoff, Methyl, Ethyl, Propyl, Butyl, Pentyl und iso-Butyl wobei Wasserstoff, Methyl und Ethyl besonders und Methyl und Ethyl ganz besonders bevorzugt sind.

Für n liegen die am meisten bevorzugten Werte im Bereich von 3 bis 20.

Auch für m gibt es besonders bevorzugte Werte, diese liegen im Bereich von 3 bis 20.

Ebenso gibt es für p besonders bevorzugte Werte, diese liegen im Bereich von 3 bis 20.

Die Summe aus n, m und p liegt besonders bevorzugt bei 3 bis 20. Dabei können die einzelnen Alkoxideinheiten in verschiedener Weise angeordnet sein, z. B. statistisch, in Blockform oder mit einem Gradienten. Besonders bevorzugt sind Verfahren, bei denen zuerst ein höheres Oxid, wie z. B. Propylenoxid und dann EO verwendet wird.

Es können in dem erfindungsgemäßen Verfahren neben verschiedenen Alkylenoxiden auch verschiedene Alkoxylate a, b, c, ..., z gleichzeitig oder zeitversetzt eingesetzt werden. So können vorteilhaft Verbindungen eingesetzt werden, bei denen Ra = C_{12, 14} bis C₂₀ und Rb = C₂ bis C₁₀ ist.

Als Base kommen grundsätzlich alle Verbindungen in Frage, die in der Lage sind, das eingesetzte Alkoxylat zu deprotonieren. Hierzu gehören auch die Basen im Sinne der Bjerrumschen Säure-Base Definition. Es gibt dabei bestimmte bevorzugte Basen und so stellt ein Verfahren, bei dem die Base ausgewählt ist aus der Gruppe bestehend aus NaOH, KOH, Mg(OH)₂, Ca(OH)₂, Ammoniumhydroxid, Cholinhydroxid und Hydrotalcit, eine besonders bevorzugte Ausführungsform der vorliegenden Erfindung dar. Hydrotalcit ist besonders bevorzugt, wenn das Produkt enge Molmassenverteilungen aufweisen soll.

Die Zugabe der Base zu der Reaktionsmischung erfolgt dabei während der Reaktion entweder absatzweise oder kontinuierlich. Das bedeutet, dass die Base oder Teile der Base der Reaktionsmischung zu einem Zeitpunkt zugesetzt werden, an dem Reaktionsbedingungen vorliegen.

Eine bevorzugte Ausführungsform ist danach ein Verfahren, bei dem die Base an 2 bis 10000 Zeitpunkten der Reaktionsmischung zugesetzt wird. Die Zugabe erfolgt vorzugsweise an 3 bis 1000, besonders bevorzugt an 4 bis 100 Zeitpunkten und ganz besonders bevorzugt an 5 bis 20 Zeitpunkten.

Eine andere bevorzugte Ausführungsform stellt ein Verfahren dar, bei dem die Base über einen Zeitraum von mehr als 30 Minuten der Reaktionsmischung zugesetzt wird. Dabei ist die Zugabe über einen Zeitraum im Bereich von mehr als 2 h bis 10 h, bevorzugt im Bereich von 5 h bis 9 h und ganz besonders bevorzugt im Bereich von 6 h bis 8 h besonders vorteilhaft.

In beiden Fällen, also sowohl bei der kontinuierlichen als auch bei der absatzweisen Zugabe der Base, kann diese an verschiedenen Stellen in die Reaktionsmischung gegeben werden. Es ist besonders bevorzugt, wenn die Base an 2 bis 10 Stellen, vorzugsweise 3 bis 5 Stellen dem Reaktor zugegeben wird. Durch diese Vorgehensweise kann die lokale Konzentration der Base in der Reaktionsmischung noch weiter verringert und so die Bildung von Nebenprodukten weiter unterbunden werden.

Als Katalysator können sowohl als Haupt- als auch als Nebenbestandteil die Metalle der Gruppen der Gruppen 4 bis 12 also von Ti bis Zn und darunter stehende in Betracht kommen.

Das Übergangsmetall liegt in aktivierter Form als Raney-Kupfer vor. Dieses kann auch noch weiter modifiziert sein, z. B. durch eine Dotierung mit anderen Metallen. Es ist ein Vorteil des erfindungsgemäßen Verfahrens, dass eine derartige Dotierung zur Erreichung eines guten Syntheseergebnisses nicht erforderlich ist, gleichwohl kann sie zur weiteren Verbesserung des Syntheseergebnisses vorteilhaft sein. Die Herstellung von geeigneten Übergangsmetallkatalysatoren ist z. B. in EP 1 125 634 (Degussa) beschrieben, in der ein einfacher Raney-Cu -fixed bed Katalysator offenbart wird. EP 1 125 633 (Degussa) in beschreibt Raney-Cu mit verschiedenen Dotierungen [Bsp: 1% Pt, 3% Fe, 2000 ppm Cr oder 1% V auf Raney Cu], wobei gezeigt wird, dass die dotierten Katalysatoren im Vergleich zum herkömmlichen Raney-Cu (Degussa BFX 3113W) weniger deaktivieren. WO 96/01146 (Monsato) schließlich beschreibt C support mit einem Edelmetall, z. B. Pt als Ankermetall auf dem Cu aufgebracht ist. Diese Technik soll ebenfalls das Sintern des Cu verhindern. Eine besonders bevorzugte Kombination von Übergangsmetallen ist die von Kupfer und Eisen. Insbesondere bevorzugt sind DMC-Katalysatoren.

Der Katalysator verbleibt beim vorliegenden Verfahren nur in geringen Mengen im Produkt. Es ist ein Verfahren bevorzugt, bei dem das Produkt M im Bereich von 0,1 ppm bis 1%, vorzugsweise im Bereich von 1 bis 500 ppm und besonders bevorzugt im Bereich von 10 bis 50 ppm aufweist.

Das erfindungsgemäße Verfahren kann erfreulicherweise auch im großen Maßstab durchgeführt werden. So ist ein Verfahren bevorzugt, bei dem mehr als 1 kg, vorzugsweise mehr als 10 kg, besonders bevorzugt mehr als 100 kg und ganz besonders bevorzugt mehr als 1 t Alkoxylat verwendet wird.

Das erfindungsgemäße Verfahren, bei dem die Reaktionstemperatur im Bereich von 140 °C bis 220 °C liegt, ist bevorzugt. Noch bevorzugter sind Verfahren, die im Temperaturbereich von 160 bis 200 °C und insbesondere im Bereich von 160 bis 190 °C, wie z. B. bei 180 °C durchgeführt werden.

Als Lösungsmittel für das erfindungsgemäße Verfahren kommen grundsätzlich alle Lösungsmittel in Frage, die unter den Reaktionsbedingungen stabil sind und weder mit den Reaktanden noch mit den Produkten reagieren. Bevorzugt sind daher Lösungsmittel ausgewählt aus der nachfolgenden Liste.

**Name**

| NMP |
|---|
| Diethylenglykoldimethylether |
| Triethylenglykoldimethylether |
| Tetraethylenglykoldimethylether |
| Diethylenglykoldiethylether |
| [2-(2-Methoxy-ethoxy)-ethoxy]-säure Natriumsalz |
| {2-[2-(2-Methoxyethoxy)-ethoxy]-ethoxy}-acetic acid |
| Decan |
| Decalin |
| Dodecan |
| Weißöl |
| 1,1,3,3-Tetramethylharnstoff |
| Tributylamin |
| γ-Butyrolacton |
| Undecan |
| tert. Butylbenzol |
| Dipropylcarbonat |
| 2,5-Dimethylpyrrol |
| 2,6-Dimethylanisol |
| 3,4-Dimethylanisol |
| 1,2-Dimethylimidazol |
| 4-tert.-Butyl1,2-dimethylbenzol |

Und besonders bevorzugt sind die folgenden Lösungsmittel bzw. Suspensionsmittel: N-Methylpyrrolidon, Triethylenglykoldimethylether, [2-(2-Methoxy-ethoxy)-ethoxy]-säure Natriumsalz und Weißöl.

Dabei gibt es auch einen bevorzugten Druckbereich. Es ist ein Verfahren bevorzugt, bei dem der Druck im Reaktionsgefäß weniger als 10 bar, vorzugsweise weniger als 5 bar, besonders bevorzugt weniger als 2 bar beträgt. Ein besonderer Vorteil des erfindungsgemäßen Verfahrens ist, dass es auch unter Normaldruck durchgeführt werden kann, hierbei handelt es sich um die am meisten bevorzugte Ausführungsform.

Ebenso geschützt sind die nach diesem Verfahren herstellbaren und nach diesem Verfahren hergestellten Ethercarboxylate.

Die Verwendung dieser Ethercarboxylate in Reinigungs-, Pflanzenschutz- und Kosmetikanwendungen sowie als Löslichkeitsvermittler stellt einen weiteren Gegenstand der vorliegenden Erfindung dar.

Auch Zusammensetzungen, die nach diesem Verfahren hergestellte Ethercarboxylate umfassen, sind Gegenstand der vorliegenden Erfindung.

Weitere gängige Bestandteile derartiger Zusammensetzungen sind z. B. in der WO 2008 / 071582 genannt.

Die vorliegende Erfindung wird durch die folgenden, den Bereich der Erfindung nicht einschränkenden Beispiele näher erläutert:

### Beispiele:

### Beispiel 1: Synthese des nichtionischen Ethoxilates (Tensid A)

300 g eines C_{12, 14}-Alkoholgemisches aus C₈ < 0,3 Gew.-%, C₁₀ < 1,0 Gew.-%, C₁₂ > 65,0 Gew.-%, C₁₄ 21,0 bis 28,0 Gew.-%, C₁₆ 4,0 bis 8,0 Gew.-%, C₁₈ < 0,5 Gew.-%, zum Beispiel Radianol C₁₂-C₁₆ 1726 der Fa. Oleon, wurden mit 2,6 g KOH (45 Gew.-% in Wasser) vorgelegt und zusammen bei 80 °C und reduziertem Druck (ca. 20 mbar) entwässert. Dann wurden bei 150 °C 462 g Ethylenoxid zudosiert und bei dieser Temperatur abreagiert. Das Ende der Reaktion wurde über den Druckabfall ermittelt. Nach Spülen mit Inertgas und Abkühlen auf Raumtemperatur wurde der Katalysator durch Zugabe von 1,25 g konzentrierter Essigsäure neutralisiert.

### Beispiel 2: Oxidative Dehydrierung unter Normaldruck im semi-batch Verfahren

In einem 2 I Mehrhalskolben mit Innenthermometer, Tropftrichter und Destillationsbrücke mit Kolonne wurden 498 g Tensid A sowie 30 g Raney-Kupfer vorgelegt und unter Rühren auf 180 °C erhitzt. Über einen Zeitraum von 8 h wurden 152 g Natronlauge (25 %ig) zugetropft und das Wasser aus dem Reaktionsgemisch abdestilliert. Nach der vollständigen Zugabe der Natronlauge wurde noch weitere 30 Minuten nachgerührt. Nach Abkühlen auf 140 °C wurde über eine Fritte filtriert und der Austrag analysiert.

Titration starke Base:
7 mg KOH/g

Titration schwache Base:
95 mg KOH/g

OH-Zahl Analytik:
Edukt: 119 mg KOH/g
Austrag:13 mg KOH/g, korrigiert mit starker Base 6 mgKOH/g; daraus ergab sich ein Umsatz von 95 %.

NMR (DMSO, CDCl₃ = 1/1); ¹H-NMR, ¹³C entkoppelt:
δ = 0,85 (t, 3H, -CH₃), 1,25 (s, 20H, CH₂), 1,55 (m, 2H, -CH₂CH₂-O), 3,4 (m, 2H, CH₂-O), 3,6 (m, 24H, O-CH₂-CH₂-O sowie CH₂-OH vom Edukt), 3,75 (s, 1,77H, CH₂-COO). Daraus ergab sich ein Umsatz von 89 %.

HPLC: Die Probe wurde in einem System getrennt, das für die Bestimmung von freiem Polyethylenglykol in nichtionischen Tensiden entwickelt wurde. Das Prinzip der Analyse beruht darauf, dass Moleküle mit aliphatischen Resten an einer Umkehrphase zurückgehalten werden, während polare Substanzen die Säule ohne Retention passieren. Mit Hilfe eines Schaltventils wurde die nicht retardierte Fraktion auf eine Größenausschluss-Säule transferiert, auf der die polymeren Bestandteile von niedermolekularen Nebenkomponenten abgetrennt wurden. Der Gehalt an Spaltprodukten bei der untersuchten Probe lag unter 0,5 g/100 g.

### Beispiel 3: Oxidative Dehydrierung unter Druck im batch-Verfahren

In einen 1, 2 I Autoklaven mit Druckhaltung bei 15 bar wurden 249 g Tensid A zusammen mit 15 g Raney-Kupfer (Degussa) und 76 g Natronlauge (25%ig) vorgelegt und unter Rühren für 10 h auf 180 °C erwärmt. Nach Abtrennung des Katalysators durch Filtration wurde die organische Phase separiert und mittels Titration auf schwache Base (6 mg KOH/g) untersucht bzw. mit Hilfe einer OH-Zahl-Messung (113 mg KOH/g) der Umsatz (6 %) bestimmt.

### Beispiel 4: Oxidative Dehydrierung unter Druck im semi-batch Verfahren

In einen 1,2 I Autoklaven mit Druckhaltung bei 15 bar wurden 249 g Tensid A zusammen mit 15 g Raney-Kupfer (Degussa) vorgelegt und unter Rühren auf 180 °C erwärmt. Im Verlauf von 7 h wurden mittels einer HPLC Pumpe insgesamt 76 g Natronlauge (25%ig) in den Autoklaven dosiert und abschließend noch 30 Minuten nachgerührt. Nach Abtrennung des Katalysators durch Filtration wurde die organische Phase separiert und mittels Titration auf schwache Base (9 mg KOH/g) untersucht bzw. mit Hilfe einer OH-Zahl-Messung (115 mg KOH/g) der Umsatz (3,5%) bestimmt.

## Patentansprüche

1. Verfahren zur Herstellung von Ethercarboxylat der Formel III 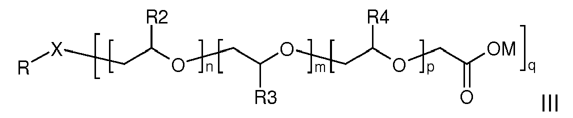 man Verbindung der Formel IV 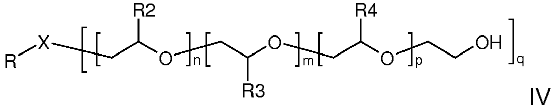 wobei jeweils unabhängig voneinander
R ein C₁- bis C₅₀-Alkyl, C₁- bis C₅₀-Alkenyl, C₁- bis C₅₀- Alkinyl, C₆- bis C₅₀-Aryl, C₆- bis C₅₀-Alkylaryl, H oder ein Metall ist
X O, COO, CH₂-NH-O für q = 1 oder N, 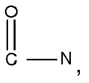 N(CH₂)ₜO für q = 2,
R2 H oder C₁- bis C₁₀-Alkyl ist,
R3 H oder ein C₁- bis C₁₀-Alkyl ist,
R4 H oder ein C₁- bis C₁₀-Alkyl ist,
M H oder ein Metall, Ammonium oder organische Base ist,
n eine Zahl von 0 bis 50 ist,
m eine Zahl von 0 bis 40 ist,
p eine Zahl von 0 bis 40 ist,
q eine Zahl von 1 bis 2 ist,
t eine Zahl von 1 bis 10 ist,
mit der Maßgabe, dass n + m + p mindestens 1 ist
mit einer Base unter Verwendung eines Übergangsmetallkatalysators, der als Raney-Kupfer vorliegt, umsetzt und wobei der Druck im Reaktionsgefäß weniger als 10 bar beträgt.

2. Verfahren nach Anspruch 1, **dadurch gekennzeichnet, dass** man Verbindung der Formel VI 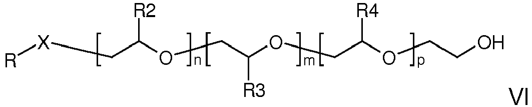 wobei jeweils unabhängig voneinander
R ein C₁- bis C₅₀-Alkyl, C₁- bis C₅₀-Alkenyl, C₁- bis C₅₀- Alkinyl, C₆- bis C₅₀-Aryl, C₆- bis C₅₀-Alkylaryl, H oder ein Metall ist,
X O ist,
R2 H oder C₁- bis C₁₀-Alkyl ist,
R3 H oder ein C₁- bis C₁₀-Alkyl ist,
R4 H oder ein C₁- bis C₁₀-Alkyl ist,
M H oder ein Metall ist,
n eine Zahl von 0 bis 50 ist,
m eine Zahl von 0 bis 40 ist,
p eine Zahl von 0 bis 40 ist,
mit der Maßgabe, dass n + m + p mindestens 1 ist
mit einer Base unter Verwendung eines Übergangsmetallkatalysators, der als Raney-Kupfer vorliegt, umsetzt und wobei der Druck im Reaktionsgefäß weniger als 10 bar beträgt.

3. Verfahren nach Anspruch 1 oder 2, bei dem unabhängig voneinander
R ein C₁- bis C₅₀-Alkyl, C₁- bis C₅₀-Alkenyl, C₁- bis C₅₀- Alkinyl, C₆- bis C₅₀-Aryl, C₆- bis C₅₀-Alkylaryl, H oder ein Metall ist X O, 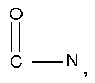 COO, CH₂-NH-O, N(CH₂)ₜO ist
R2 H oder ein C₂- bis C₈-Alkyl ist,
R3 H oder ein C₂- bis C₈-Alkyl ist,
R4 H oder ein C₂- bis C₈-Alkyl ist,
n eine Zahl von 1 bis 30 ist,
m eine Zahl von 1 bis 20 ist,
p eine Zahl von 1 bis 20 ist,
n + m + p mindestens 2 ist und
t eine Zahl von 1 bis 10 ist.

4. Verfahren nach Anspruch 1 bis 2, bei dem unabhängig voneinander X O, COO ist,
R ein C₂- bis C₁₀-Alkyl ist,
R2 H oder ein C₃- bis C₅-Alkyl ist,
R3 H oder ein C₃- bis C₅-Alkyl ist,
R4 H oder ein C₃- bis C₆-Alkyl ist, n eine Zahl von 1 bis 18 ist,
m eine Zahl von 1 bis 12 ist,
m eine Zahl von 1 bis 12 ist, n + m + p mindestens 5 ist.

5. Verfahren nach einem der vorhergehenden Ansprüche, bei dem die Base ausgewählt ist aus der Gruppe bestehend aus NaOH, KOH, Mg(OH)₂, Ca(OH)₂, Ammoniumhydroxid, Cholinhydroxid und Hydrotalcit.

6. Verfahren nach einem der vorhergehenden Ansprüche, bei dem die Base an 2 bis 10000 Zeitpunkten der Reaktionsmischung zugesetzt wird.

7. Verfahren nach einem der vorhergehenden Ansprüche, bei dem die Base über einen Zeitraum von mehr als 30 Minuten der Reaktionsmischung zugesetzt wird.

8. Verfahren nach einem der vorhergehenden Ansprüche, bei dem die Reaktionstemperatur im Bereich von 140 °C bis 220 °C liegt.

9. Verfahren nach einem der vorhergehenden Ansprüche, **dadurch gekennzeichnet, dass** R für Alkali oder Erdalkali steht.

10. Verfahren nach einem der vorhergehenden Ansprüche, **dadurch gekennzeichnet, dass** M für Alkali oder Erdalkali steht.

## Claims

1. A process for preparing an ether carboxylate of formula III 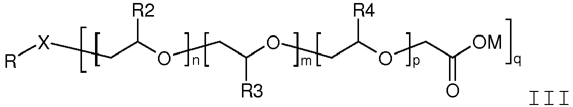 reacting the compound of formula IV 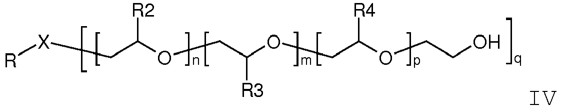 where independently
R is C₁- to C₅₀-alkyl, C₁- to C₅₀-alkenyl, C₁- to C₅₀-alkynyl, C₆- to C₅₀-aryl, C₆- to C₅₀-alkylaryl, H or a metal,
X is O, COO, CH₂-NH-O for q = 1 or N, 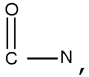 N(CH₂)ₜ O for q = 2,
R2 is H or C₁- to C₁₀-alkyl,
R3 is H or C₁- to C₁₀-alkyl,
R4 is H or C₁- to C₁₀-alkyl,
M is H or a metal, ammonium or organic base,
n is from 0 to 50,
m is from 0 to 40,
p is from 0 to 40,
q is from 1 to 2,
t is from 1 to 10,
provided that n + m + p is at least 1,
with a base by using a transition metal catalyst which is present as Raney copper, wherein the pressure in the reaction vessel is less than 10 bar.

2. The process according to claim 1 wherein the compound of formula VI 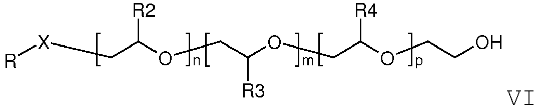 where independently
R is C₁- to C₅₀-alkyl, C₁- to C₅₀-alkenyl, C₁- to C₅₀-alkynyl, C₆- to C₅₀-aryl, C₆- to C₅₀-alkylaryl, H or a metal,
X is 0,
R2 is H or C₁- to C₁₀-alkyl,
R3 is H or C₁- to C₁₀-alkyl,
R4 is H or C₁- to C₁₀-alkyl,
M is H or a metal,
n is from 0 to 50,
m is from 0 to 40,
p is from 0 to 40,
provided that n + m + p is at least 1,
is reacted with a base by using a transition metal catalyst which is present as Raney copper, and wherein the pressure in the reaction vessel is less than 10 bar.

3. The process according to claim 1 or 2 wherein independently
R is C₁- to C₅₀-alkyl, C₁- to C₅₀-alkenyl, C₁- to C₅₀-alkynyl, C₆- to C₅₀-aryl, C₆- to C₅₀-alkylaryl, H or a metal,
X is O, 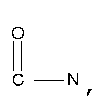 COO, CH₂-NH-O, N(CH₂)ₜO
R2 is H or C₂- to C₈-alkyl,
R3 is H or C₂- to C₈-alkyl,
R4 is H or C₂- to C₈-alkyl,
n is from 1 to 30,
m is from 1 to 20,
p is from 1 to 20,
n + m + p is at least 2 and
t is from 1 to 10.

4. The process according to either of claims 1 and 2 wherein independently
X is O or COO,
R is C₂- to C₁₀-alkyl,
R2 is H or C₃- to C₅-alkyl,
R3 is H or C₃- to C₅-alkyl,
R4 is H or C₃- to C₆-alkyl,
n is from 1 to 18,
m is from 1 to 12,
m is from 1 to 12,
n + m + p is at least 5.

5. The process according to any preceding claim wherein the base is selected from the group consisting of NaOH, KOH, Mg(OH)₂, Ca(OH)₂, ammonium hydroxide, choline hydroxide and hydrotalcite.

6. The process according to any preceding claim wherein the base is added to the reaction mixture at 2 to 10 000 times.

7. The process according to any preceding claim wherein the base is added to the reaction mixture over a period of more than 30 minutes.

8. The process according to any preceding claim wherein the reaction temperature is in the range from 140°C to 220°C.

9. The process according to any preceding claim wherein R is alkali or alkaline earth.

10. The process according to any preceding claim wherein M is alkali or alkaline earth.

## Revendications

1. Procédé de fabrication d'éther-carboxylate de formule III 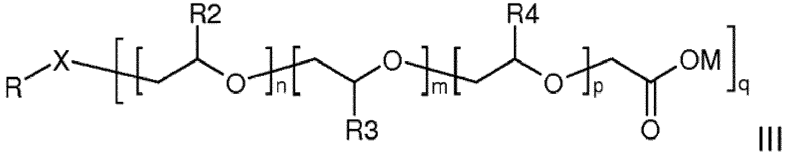 un composé de formule IV 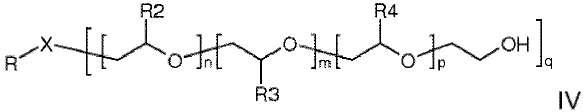 dans laquelle, à chaque fois indépendamment les uns des autres,
R représente un alkyle en C₁ à C₅₀, un alcényle en C₁ à C₅₀, un alcinyle en C₁ à C₅₀, un aryle en C₆ à C₅₀, un alkylaryle en C₆ à C₅₀, H ou un métal,
X représente O, COO, CH₂-NH-O pour q = 1, ou N, 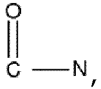
N (CH₂) ₜO pour q = 2,
R2 représente H ou alkyle en C₁ à C₁₀,
R3 représente H ou alkyle en C₁ à C₁₀,
R4 représente H ou alkyle en C₁ à C₁₀,
M représente H ou un métal, l'ammonium ou une base organique,
n représente un nombre de 0 à 50,
m représente un nombre de 0 à 40,
p représente un nombre de 0 à 40,
q représente un nombre de 1 à 2,
t représente un nombre de 1 à 10,
à condition que n + m + p soit au moins 1,
est mis en réaction avec une base en utilisant un catalyseur de métal de transition, qui se présente sous la forme de cuivre de Raney, et la pression dans le récipient de réaction étant inférieure à 10 bar.

2. Procédé selon la revendication 1, **caractérisé en ce qu'**un composé de formule VI 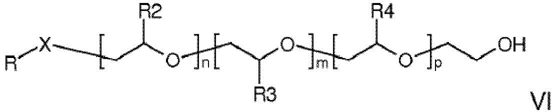 dans laquelle, à chaque fois indépendamment les uns des autres,
R représente un alkyle en C₁ à C₅₀, un alcényle en C₁ à C₅₀, un alcinyle en C₁ à C₅₀, un aryle en C₆ à C₅₀, un alkylaryle en C₆ à C₅₀, H ou un métal,
X représente O,
R2 représente H ou alkyle en C₁ à C₁₀,
R3 représente H ou alkyle en C₁ à C₁₀,
R4 représente H ou alkyle en C₁ à C₁₀,
M représente H ou un métal,
n représente un nombre de 0 à 50,
m représente un nombre de 0 à 40,
p représente un nombre de 0 à 40,
à condition que n + m + p soit au moins 1,
est mis en réaction avec une base en utilisant un catalyseur de métal de transition, qui se présente sous la forme de cuivre de Raney, et la pression dans le récipient de réaction étant inférieure à 10 bar.

3. Procédé selon la revendication 1 ou 2, selon lequel, indépendamment les uns des autres
R représente un alkyle en C₁ à C₅₀, un alcényle en C₁ à C₅₀, un alcinyle en C₁ à C₅₀, un aryle en C₆ à C₅₀, un alkylaryle en C₆ à C₅₀, H ou un métal,
X représente O, 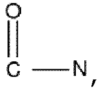 COO, CH₂-NH-O, N(CH₂)ₜO_{,}
R2 représente H ou alkyle en C₂ à C₈,
R3 représente H ou alkyle en C₂ à C₈,
R4 représente H ou alkyle en C₂ à C₈,
n représente un nombre de 1 à 30,
m représente un nombre de 1 à 20,
p représente un nombre de 1 à 20,
n + m + p sont au moins 2, et
t représente un nombre entier de 1 à 10.

4. Procédé selon l'une des revendications 1 ou 2, dans lequel, indépendamment les uns des autres,
X représente 0, COO,
R représente un alkyle en C2 à C10,
R2 représente H ou un alkyle en C3 à C5,
R3 représente H ou un alkyle en C3 à C5,
R4 représente H ou un alkyle en C3 à C6,
n représente un nombre de 1 à 18,
m représente un nombre de 1 à 12,
m représente un nombre de 1 à 12,
n + m + p représente au moins 5.

5. Procédé selon l'une quelconque des revendications précédentes, dans lequel la base est choisie dans le groupe constitué par NaOH, KOH, Mg(OH)₂, Ca (OH)₂, l'hydroxyde d'ammonium, l'hydroxyde de choline et l'hydrotalcite.

6. Procédé selon l'une quelconque des revendications précédentes, dans lequel la base est ajoutée au mélange réactionnel en 2 à 10 000 instants.

7. Procédé selon l'une quelconque des revendications précédentes, dans lequel la base est ajoutée au mélange réactionnel en une durée de plus de 30 minutes.

8. Procédé selon l'une quelconque des revendications précédentes, selon lequel la température de réaction se situe dans la plage allant de 140 °C à 220 °C.

9. Procédé selon l'une quelconque des revendications précédentes, **caractérisé en ce que** R représente un alcali ou un alcalino-terreux.

10. Procédé selon l'une quelconque des revendications précédentes, **caractérisé en ce que** M représente un alcali ou un alcalino-terreux.
